(19) Europäisches Patentamt
European Patent Office
Office européen des brevets

(11) Veröffentlichungsnummer: **0 131 943**
**A2**

(12) # EUROPÄISCHE PATENTANMELDUNG

(21) Anmeldenummer: **84108317.3**

(22) Anmeldetag: **14.07.84**

(51) Int. Cl.[4]: **C 07 D 233/36**
A 61 K 31/415

(30) Priorität: **18.07.83 DE 3325875**

(43) Veröffentlichungstag der Anmeldung:
**23.01.85 Patentblatt 85/4**

(84) Benannte Vertragsstaaten:
**AT BE CH DE FR IT LI LU NL SE**

(71) Anmelder: **BOEHRINGER INGELHEIM KG**

**D-6507 Ingelheim am Rhein(DE)**

(72) Erfinder: **Mentrup, Anton, Dr.**
**Steinernstrasse 25**
**D-6503 Mainz-Kastel(DE)**

(72) Erfinder: **Schromm, Kurt, Dr.**
**In der Dörrwiese 35**
**D-6507 Ingelheim am Rhein(DE)**

(72) Erfinder: **Renth, Ernst-Otto, Dr.**
**Frankenstrasse 11**
**D-6507 Ingelheim am Rhein(DE)**

(72) Erfinder: **Muacevic, Gojko, Dr.**
**In der Dörrwiese 13**
**D-6507 Ingelheim am Rhein(DE)**

(72) Erfinder: **Fügner, Armin, Dr.**
**Im Hippel 31**
**D-6535 Gau-Algesheim(DE)**

(54) **Neue 1-Phenylimidazolidin-2-on-derivate, ihre Herstellung und Verwendung.**

(57) Die neuen Verbindungen der Formel

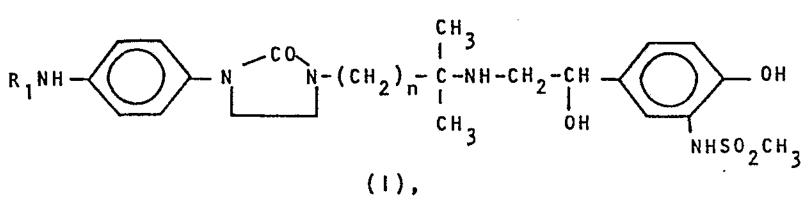

(I),

die nach üblichen Verfahren hergestellt werden können, sind als Arzneistoffe geeignet und können insbesondere als Broncholytika verwendet werden.

EP 0 131 943 A2

Croydon Printing Company Ltd

Die Erfindung betrifft neue 1-Phenylimidazolidin-2-on-Derivate, ihre Herstellung nach an sich bekannten Verfahren und ihre Verwendung als Arzneistoffe.

Die neuen Verbindungen haben die Formel

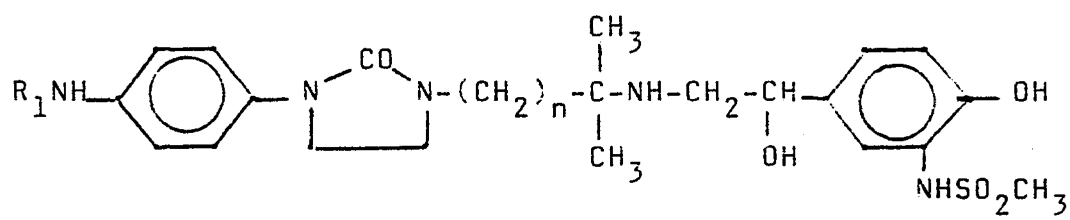

(I),

in der

n für 1 oder 2,

$R_1$ für Wasserstoff, $COR_2$ oder $SO_2R_3$,

$R_2$ für Wasserstoff, $C_1$-$C_4$-Alkyl, $C_1$-$C_4$-Alkoxy oder NH-($C_1$-$C_4$-Alkyl) und

$R_3$ für $C_1$-$C_4$-Alkyl steht.

Sie können in Form von Gemischen der Enantiomeren oder auch in Form der reinen Enantiomeren vorliegen, jeweils als freie Basen oder als Salze mit Säuren.

Die in der Definition genannten Alkyl- und Alkoxygruppen können geradkettig oder verzweigt sein. Bevorzugt enthalten diese Gruppen 1 bis 2 C-Atome. Die Gruppe $COR_2$ steht vor allem für CHO, $CH_3CO$, $CH_3OCO$, $C_2H_5OCO$, die Gruppe $SO_2R_3$ insbesondere für $SO_2CH_3$.

Aus der deutschen Offenlegungsschrift 2 609 645 sind verwandte Verbindungen bekannt, doch zeigen sie keine so günstige Wirkungsverhältnisse wie die Verbindungen der obigen Formel I.

Die neuen Verbindungen können nach den folgenden Verfahren erhalten werden.

1. Reduktive Alkylierung eines Phenylglyoxals bzw. eines entsprechenden Halbacetals der Formel

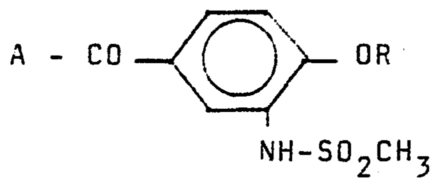 (II),

in der A für CHO oder 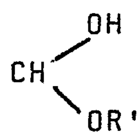 (R': H oder $C_1$-$C_4$-Alkyl) und
R für eine hydrolytisch oder hydrogenolytisch abspaltbare Schutzgruppe steht, mit einer Verbindung der Formel

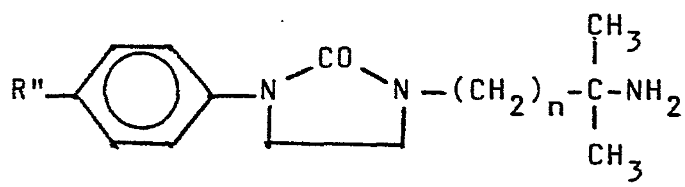 (III)

in der R" für $NH_2$, $NHCOR_2$, $NHSO_2R_3$ oder $NO_2$,
n für 1 oder 2 steht.

Als Reduktionsmittel für die reduktive Aminierung werden komplexe Hydride, vorzugsweise Natriumborhydrid, oder Wasserstoff in Gegenwart von Hydrierungskatalysatoren, etwa Platin, Palladium, Nickel, verwendet.

Als Schutzgruppe R dienen vor allem Benzyl, das hydrogenolytisch abgespalten wird, und Acylreste, insbesondere von niederen Carbonsäuren, die durch alkalische oder saure Hydrolyse entfernt werden. Die Schutzgruppen können während oder nach der reduktiven Alkylierung abgespalten werden.

Hat R" die Bedeutung "Nitrogruppe", erfolgt die Reduktion zur Aminogruppe entweder bei der reduktiven Aminierung oder anschließend durch eine katalytische Hydrierung.

Die eventuell bei der reduktiven Aminierung auftretende Zwischenverbindung

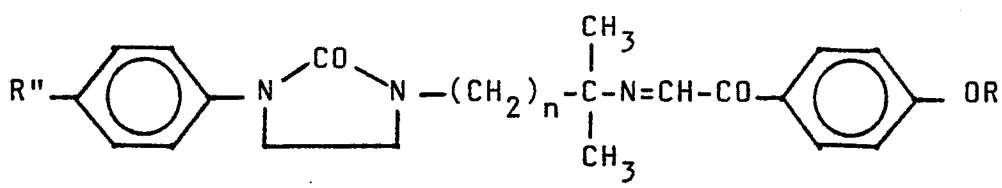

(IV)

kann auch isoliert und dann reduziert werden.

Die Umsetzung nach dem vorstehenden Verfahren erfolgt in Lösungsmitteln, die unter den Reaktionsbedingungen inert sind, z.B. in niederen Alkoholen, wie Methanol, Ethanol, Propanol. Bei der Verwendung von Hydriden als Reduktionsmittel wird vorzugsweise gekühlt, die Hydrierung kann auch bei erhöhter Temperatur erfolgen.

Die Ausgangsstoffe II und III sind bekannt oder können nach üblichen Verfahren hergestellt werden.

2. Reduktion eines Aminoketons der Formel

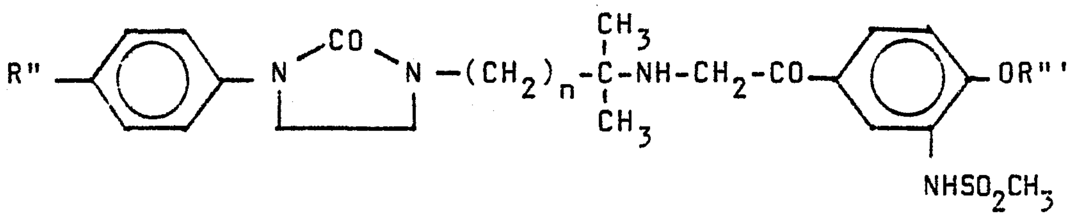

(V)

in der n und R" die obige Bedeutung haben und R"' für Wasserstoff, $CH_2$-Aryl, CO-($C_1$-$C_4$-Alkyl) oder CO-Aryl (Aryl vorzugsweise gegebenenfalls substituiertes Phenyl) steht.

Als Reduktionsmittel dienen beispielsweise Wasserstoff und Hydrierungskatalysatoren wie Platin, Palladium, Nickel oder komplexe Hydride, insbesondere Natriumborhydrid.

Hat R" die Bedeutung "Nitrogruppe", erfolgt die Reduktion zur Aminogruppe entweder gleichzeitig mit der katalytischen Hydrierung der Aminoketone oder durch eine katalytische Hydrierung nach der Reduktion des Aminoketons mit Hydriden.

Wenn R"' eine Schutzgruppe darstellt, wird diese entweder bei oder nach der Reduktion der Ketogruppe abgespalten. Dabei erfolgt die Entfernung der Arylmethylgruppe durch katalytische Hydrierung, die Abspaltung der Acylgruppe durch eine saure oder basische Verseifung.

Die Ausgangsstoffe V können nach üblichen Verfahren hergestellt werden.

3. Abspaltung der Schutzgruppe(n) aus einer Verbindung der Formel

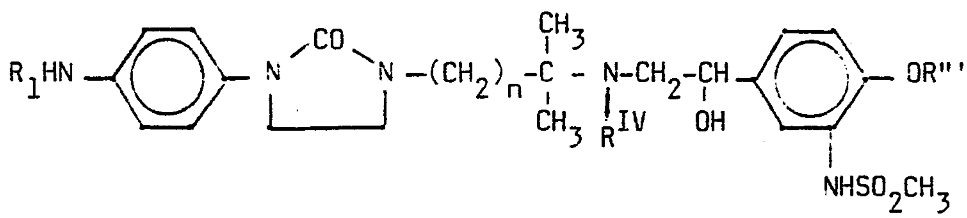

(VI),

in der n, $R_1$ und R"' die obige Bedeutung haben und $R^{IV}$ Wasserstoff oder eine hydrogenolytisch abspaltbare Gruppe ist, wobei mindestens einer der Reste R"' und $R^{IV}$ eine abzuspaltende Gruppe ist.

Falls $R^{III}$ eine hydrogenolytisch abspaltbare Gruppe ist, wird sie durch katalytische Hydrierung in Gegenwart eines Platin-, Palladium- oder Nickel-Katalysators entfernt. Ebenso wird $R^{IV}$ entfernt, wenn es eine hydrogenolytisch abspaltbare Gruppe darstellt. Ist R"' eine hydrolytisch abspaltbare Gruppe, so erfolgt die Abspaltung durch saure oder basische Verseifung.

Die Ausgangsstoffe der Formel VI werden nach üblichen Verfahren gewonnen.

Gewünschtenfalls werden die nach den Verfahren 1. bis 3. hergestellten Verbindungen, soweit sie als Basen anfallen, in Säureadditionssalze, soweit sie als Säureadditionssalze anfallen, in freie Basen überführt. Reine Enentiomere oder Gemische der Enantiomeren mit unterschiedlichen Anteilen der Antipoden

können durch Auftrennung von Racematen nach üblichen Methoden, gegebenenfalls auch durch Einsatz entsprechender Vorstufen gewonnen werden.

Die erfindungsgemäßen Verbindungen sind wertvolle Arzneistoffe, besonders aufgrund ihrer lange anhaltenden oralen broncholytischen Wirkung. Bei der Anwendung als Broncholytika wirkt sich die hohe Selektivität, d.h. die nur geringe Beeinflussung der Herzfrequenz, positiv aus. Neben der Broncholyse zeigen die erfindungsgemäßen Verbindungen u.a. noch gefäßerweiternde, antidepressive und herzleistungssteigernde Wirkung.

Für die Verbindung der Formel I, in der $R_1$ $COCH_3$ und n 2 bedeutet, beträgt beispielsweise bei der intravenösen Gabe der $ED_{50}$ (Broncholyse) am Meerschweinchen die Zunahme der Herzfrequenz nur ca. 2 Schläge pro Minute. Bei höherer Dosierung, z.B. 1,0 µg/kg, steigt die Hemmung des durch Acetylcholin erzeugten Bronchospasmus auf 70 % bei einer Halbwertszeit von über 30 Minuten, während die Herzfrequenz nur um 10 Schläge pro Minute ansteigt.
Für die Anwendung werden die erfindungsgemäßen Wirkstoffe mit den in der galenischen Pharmazie üblichen Hilfsstoffen zu gebräuchlichen Arzneimittelformen verarbeitet, z.B. zu Tabletten, Dragées, Kapseln, Tinkturen, Injektionslösungen, Suppositorien, Inhalationspräparaten.

Die Einzeldosis beträgt zwischen 1 und 500 mg, vorzugsweise 2 - 200 mg; in Abhängigkeit von Applikationsform, Wirkstoff und Körpergewicht der zu behandelnden Person. Die hohen Dosierungen kommen vor allem für Retardformen in Betracht.

Nachstehend sind Beispiele für erfindungsgemäße Arzneimittelzubereitungen angegeben.

Tabletten

Zusammensetzung:

| | |
|---|---|
| Wirkstoff gemäß der Erfindung | 2 Gew.-Teile |
| Stearinsäure | 6 Gew.-Teile |
| Traubenzucker | 592 Gew.-Teile |

Die Bestandteile werden in üblicher Weise zu Tabletten von 600 mg Gewicht verarbeitet. Gewünschtenfalls kann der Wirkstoffgehalt erhöht oder vermindert und die Traubenzuckermenge entsprechend vermindert oder erhöht werden.

Suppositorien

Zusammensetzung:

| | |
|---|---|
| Wirkstoff gemäß der Erfindung | 100 Gew.-Teile |
| Laktose, gepulvert | 45 Gew.-Teile |
| Kakao-Butter | 1555 Gew.-Teile |

Die Bestandteile werden in üblicher Weise zu Suppositorien von 1,7 g Gewicht verarbeitet.

Kapseln

Zusammensetzung:

| | |
|---|---|
| Wirkstoff gemäß der Erfindung | 10 Gew.-Teile |
| Laktose | 490 Gew.-Teile |
| Maisstärke | 400 Gew.-Teile |

Je 1000 mg fein pulverisierten Mischung werden in Hartgelatinekapseln abgefüllt.

Beispiel 1

1-(3-Methansulfonamido-4-hydroxiphenyl)-2-{1,1-dimethyl-3-/3-(4-acetaminophenyl)-imidazolidin-2-on-1-yl/-propylamino}-ethanol-hydrochlorid

a) Ein Gemisch aus 35 g 1-(4-Nitrophenyl)-imidazolidin-2-on und 8,2 g Natriumhydrid in 200 ml Hexametapol wird mit einer Lösung von 38,8 g 3-Chlor-1,1-dimethyl-1-(N-benzylidenamino)propan in 40 ml Hexametapol versetzt. Man erhitzt die Mischung 3 Stunden auf 100°C, entfernt das Lösungsmittel und erhält nach Zusatz von 100 ml 2 N Salzsäure die Verbindung der Formel

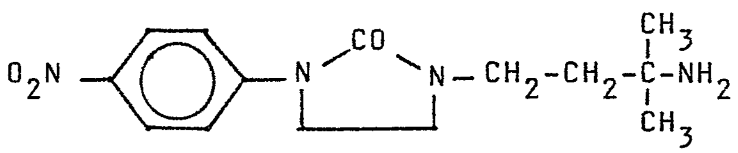

. HCl (A)

Ausbeute 40 g, Fp. 288°C.

Zur Reduktion der Nitrogruppe werden

b) 32,85 g der Verbindung A in 1500 ml Methanol in Gegenwart von 0,5 g Platinoxid bei 20°C und 5 bar hydriert. Es werden 22,2 g des A entsprechenden Aminophenylderivats als Monohydrochlorid erhalten (Verbindung B, Fp. 223-224°C).

c) 9 g der Verbindung B werden in 60 ml Eisessig mit 9 ml Acetanhydrid auf 70°C erwärmt. Nach 10 Minuten wird die Verbindung der Formel

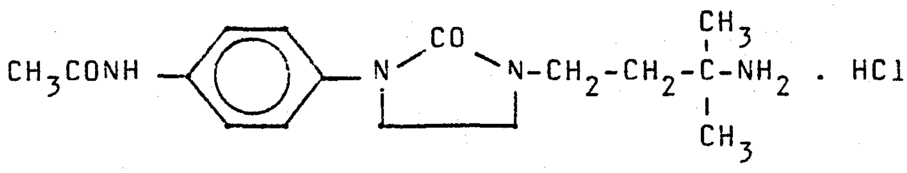 (C)

isoliert (9,5 g. Fp. 270°C). Aus dem Hydrochlorid wird durch Zugabe von wäßrigem Ammoniak die Base gewonnen. Fp. 176,5°C.

d) 3,04 g der Verbindung C werden in 100 ml Ethanol mit 4,17 g des Halbacetals der Formel

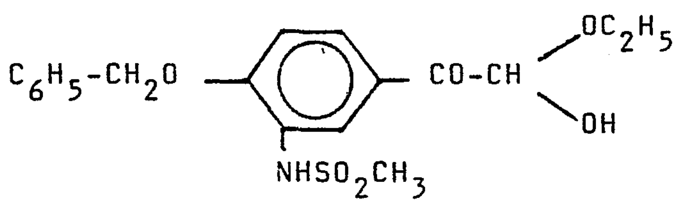 (D)

auf 70°C erwärmt und nach Stehen über Nacht bei 15 - 20°C mit 1 g Natriumborhydrid versetzt. Nach dreistündigem Rühren wird mit Salzsäure angesäuert und das isolierte Rohprodukt (7,2 g) in Acetonitril durch Zugabe von Methansulfonsäure als Salz gefällt.
Man erhält 6,6 g der Verbindung der Formel

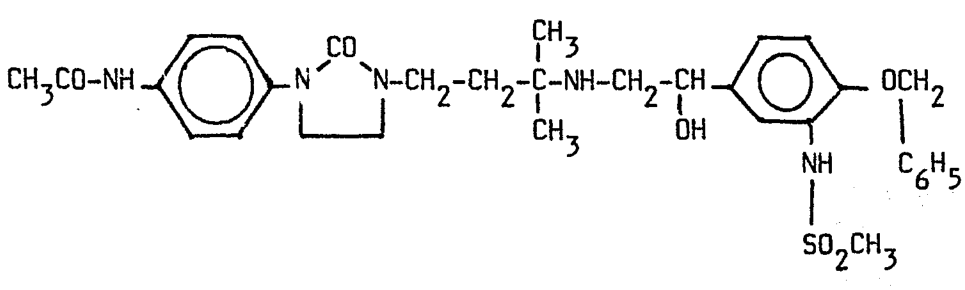 (E)

als Methansulfonat, Fp. 146°C (Verbindung E).

Analog kann die Verbindung der Formel

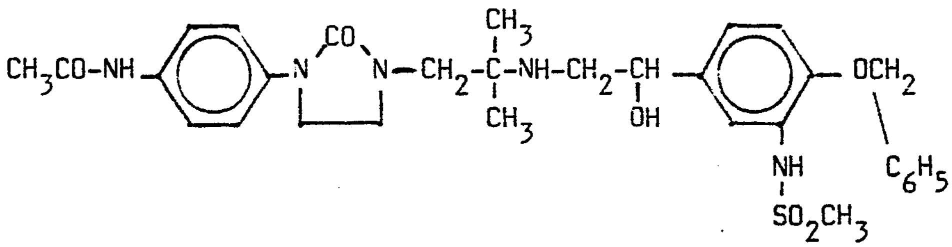

erhalten werden.

e) Zu einer Lösung von 4,2 g der Verbindung E (als Base) in 110 ml Methanol gibt man 0,8 g Palladium/Kohle (10prozentig) und hydriert bei 20°C unter Normaldruck.
Nach dem Abtrennen des Katalysators engt man die Lösung ein. Der Rückstand wird aus Ethanol umkristallisiert. Ausbeute: 3,3 g der Titelverbindung (als Base), Fp. 132°C. Durch Zugabe von etherischer Salzsäure zu der methanolischen Lösung der Base erhält man das Hydrochlorid, das mit 3 mol Wasser kristallisiert bei 137°C schmilzt.

Beispiel 2

1-(3-Methansulfonamido-4-hydroxiphenyl)-2-{1,1-dimethyl-3-[3-(4-aminophenyl)imidazolidin-2-on-1-yl]-propylamino} ethanol-hydrochlorid

Eine Lösung von 3,7 g 1-(3-Methansulfonamido-4-benzyloxiphenyl)-2- 1,1-dimethyl-3- 3-(4-nitrophenyl)imidazolidin-2-on-1-yl)-propylamino ethanol-hydrochlorid in 250 ml Methanol wird mit 0,2 g HCl in 10 ml Methanol versetzt und nach Zugabe von 0,7 g Palladium/Kohle (5 prozentig) bei 26°C und Normaldruck hydriert. Die vom Katalysator befreite Lösung wird eingeengt und die Titelverbindung in einer Ausbeute von 3,3 g erhalten. Nach dem Umkristallisieren aus Methanol liegt der Schmelzpunkt bei 225°C.

Beispiel 3

1-(3-Methansulfonamido-4-hydroxiphenyl)-2-{1,1-dimethyl-3-[3-(4-acetaminophenyl)-imidazolidin-2-on-1-yl]-propylamino}-ethanol-hydrochlorid

Eine Lösung von 0,45 g 1-(3-Methansulfonamido-4-benzyloxiphenyl)-2-{1,1-dimethyl-3-[3-(4-aminophenyl) imidazolidin-2-on-1-yl]-propylamino}ethanol-hydrochlorid in 50 ml Methanol wird in Gegenwart von 0,3 g Palladium/Kohle (5prozentig) bei 24°C und Normaldruck hydriert. Es werden 0,34 g der Titelverbindung, Fp. 225°C (aus Methanol) erhalten.

Die Verbindungen der nachstehenden Tabelle können erfindungsgemäß erhalten werden.

Verbindungen der Formel I

| Nr. | n | $R_1$ | Schmelzpunkt Base | Salz (Säure) |
|---|---|---|---|---|
| 1 | 1 | HCO | | |
| 2 | 1 | $CH_3CO$ | | |
| 3 | 1 | $C_2H_5CO$ | | |
| 4 | 1 | $n-C_3H_7CO$ | | |
| 5 | 1 | $(CH_3)_3CCO$ | | |
| 6 | 1 | $CH_3OCO$ | | |
| 7 | 1 | $C_2H_5OCO$ | | |
| 8 | 1 | $i-C_3-H_7OCO$ | | |
| 9 | 1 | $n-C_4H_9OCO$ | | |
| 10 | 1 | $CH_3SO_2$ | | |
| 11 | 1 | $C_2H_5SO_2$ | | |
| 12 | 1 | $i-C_3H_7SO_2$ | | |
| 13 | 1 | $n-C_4H_9SO_2$ | | |
| 14 | 2 | HCO | | |
| 15 | 2 | $C_2H_5CO$ | | |
| 16 | 2 | $n-C_3H_7CO$ | 186°C | 189°C (HCl) |
| 17 | 2 | $n-C_4H_9CO$ | | |
| 18 | 2 | $(CH_3)_3CCO$ | | |
| 19 | 2 | $CH_3OCO$ | | |
| 20 | 2 | $C_2H_5OCO$ | | 170°C (HCl + $1H_2O$) |
| 21 | 2 | $n-C_3H_7OCO$ | | |
| 22 | 2 | $(CH_3)_2CH-CH_2OCO$ | | |
| 23 | 2 | $CH_3SO_2$ | | 194°C (HCl) |
| 24 | 2 | $C_2H_5SO_2$ | | |
| 25 | 2 | $n-C_3H_7SO_2$ | | |
| 26 | 2 | $i-C_3H_7SO_2$ | | |
| 27 | 2 | $n-C_4H_9SO_2$ | | |
| 28 | 1 | H | | |

Patentansprüche

1. Verbindungen der Formel

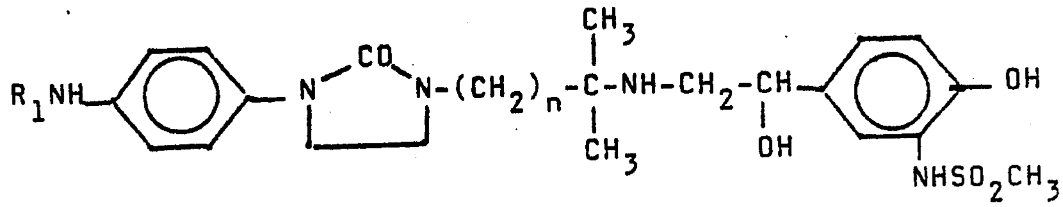

(I),

in der

n für 1 oder 2,

$R_1$ für Wasserstoff, $COR_2$ oder $SO_2R_3$,

$R_2$ für Wasserstoff, $C_1$-$C_4$-Alkyl, $C_1$-$C_4$-Alkoxy oder NH-($C_1$-$C_4$-Alkyl) und

$R_3$ für $C_1$-$C_4$-Alkyl

steht, in Form von Gemischen der Enantiomeren oder in Form der reinen Enantiomeren, als freie Basen oder Säureadditionssalze.

2. Verbindungen der Formel I, worin $R_1$ für $COR_2$ steht, wobei $R_2$ die oben angegebene Bedeutung hat.

3. Verbindungen der Formel I, worin $R_1$ für $SO_2R_3$ steht, worin $R_3$ die oben angegebene Bedeutung hat.

4. Arzneimittel, gekennzeichnet durch einen Gehalt an mindestens einer Verbindung nach Anspruch 1, 2 oder 3.

5. Verwendung von Verbindungen nach Anspruch 1, 2 oder 3 zur Behandlung von Bronchospasmen.

6. Verfahren zur Herstellung von Verbindungen nach Anspruch 1, dadurch gekennzeichnet, daß man

a) eineVerbindung der Formel

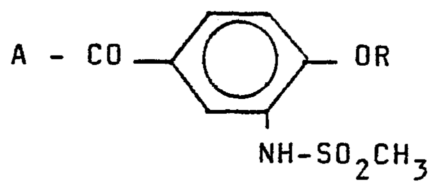

(II),

in der A für CHO oder 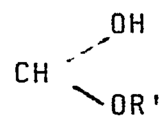

(R' H oder $C_1$-$C_4$-Alkyl) und R für eine hydrolytisch oder hydrogenolytisch abspaltbare Schutzgruppe steht, unter den Bedingungen der reduktiven Alkylierung mit einer Verbindung der Formel

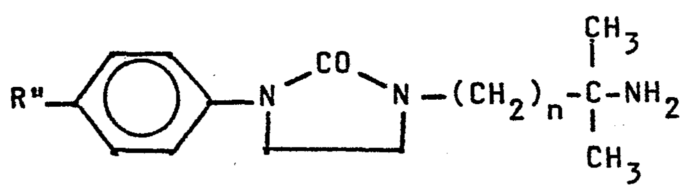

(III)

umsetzt, worin n für 1 oder 2 und R" für $NH_2$, $NHCOR_2$, $NHSO_2R_3$ oder $NO_2$ steht und gegebenenfalls anschließend die Schutzgruppe abspaltet oder daß man

b) ein Aminoketon der Formel

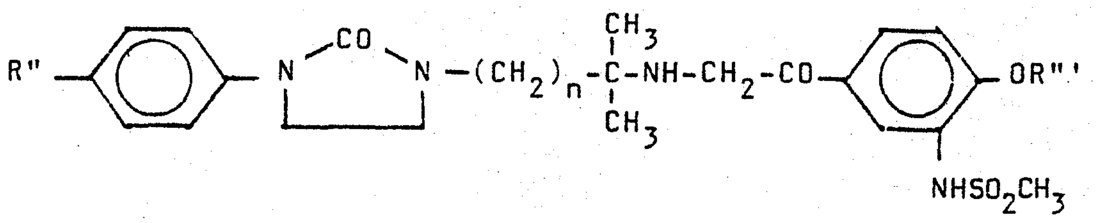

(V)

in der n und R" die obige Bedeutung haben und R"' für Wasserstoff, $CH_2$Aryl, CO-($C_1$-$C_4$-Alkyl) oder CO-Aryl steht, reduziert,

c) oder daß man aus einer Verbindung der Formel

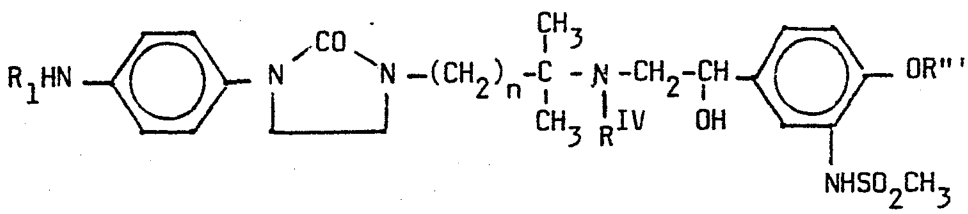

(VI),

in der n, $R_1$ und R"' die obige Bedeutung haben und $R^{IV}$ Wasserstoff oder eine hydrogenolytisch abspaltbare Gruppe ist, wobei mindestens einer der Reste R"' und $R^{IV}$ eine abzuspaltende Gruppe darstellt, diese Gruppe(n) je nach ihrer Art durch Hydrogenolyse bzw. saure oder basische Verseifung entfernt,

und daß man gewünschtenfalls die nach a) bis c) erhaltenen Produkte in die Enantiomeren auftrennt und/oder daß man sie, wenn sie als Basen anfallen, in Säureadditionssalze, wenn sie als Säureadditionssalze anfallen, in die Basen überführt.